# EUROPÄISCHE PATENTSCHRIFT

(11) **EP 0 896 080 B1**
(45) Veröffentlichungstag und Bekanntmachung des Hinweises auf die Patenterteilung: **10.09.2003**
(21) Anmeldenummer: 98114241.7
(22) Anmeldetag: 30.07.1998
(51) Int. Cl.: D04H 1/46, D04H 13/00, D04H 3/10, B32B 5/26, D04H 5/02, D04H 5/06

(54) **Verfahren zur Herstellung eines voluminösen Verbundvlieses, Vorrichtung zur Durchführung des Verfahrens und Verbundvlies nach diesem Verfahren**
Process of making a voluminous nonwoven composite fabric, fabric and apparatus for manufacturing the fabric accordingly
Méthode de fabrication d'un non-tissé composite volumineux, dispositif de réalisation et produit

(30) Priorität: 06.08.1997 DE 19733933; 16.08.1997 DE 19735667
(43) Veröffentlichungstag der Anmeldung: 10.02.1999
(73) Patentinhaber: Fleissner GmbH & Co. Maschinenfabrik, 63329 Egelsbach (DE)
(72) Erfinder: Fleissner, Gerold, 6300 Zug (CH)
(74) Vertreter: Neumann, Gerd, Dipl.-Ing.

(56) Entgegenhaltungen:
- EP-A- 0 171 806
- EP-A- 0 586 924
- WO-A-96/34136
- DE-A- 19 500 669
- DE-A- 19 522 763
- US-A- 4 514 455
- US-A- 4 731 277
- US-A- 5 413 849

## Beschreibung

Nach der DE-A-195 00 669 ist es bekannt, ein voluminöses Vlies mit der hydrodynamischen Vernadelung ohne Verwendung von Bindemitteln dadurch zu erzeugen, dass nur ein geringer Wasserdruck beim Vernadeln verwendet wird. Dagegen offenbart die DE-A-195 22 763 ein Verfahren, bei dem ein wenn auch geringeres Volumen dadurch aufrechterhalten bleibt, dass ein Krempelfaservlies auf ein verfestigtes Trägervlies abgelegt wird, das dann zur Verfestigung vemadelt und auch mit dem Trägervlies verbunden wird.

Zur Herstellung eines voluminösen Vlieses ist es durch die US-A-4 118 531 weiterhin bekannt, gekräuselte Fasern einzusetzen. Dazu werden die gekräuselten Fasern in einem Strom gemeinsam mit anderen glatten Fasern abgelegt. Da aber eine Verfestigung eines solchen Vlieses notwendig ist, muss das Vlies mechanisch verdichtet werden, womit es einen wesentlichen Teil seines Volumens wieder verliert.

Durch die US-A-5 108 820 ist es bekannt, zur Herstellung eines voluminösen Vlieses Bikomponentenfasern einzusetzen, deren Komponenten bei Erhitzung unterschiedlich schrumpfen, womit die Faser sich dreidimensional kräuselt. Auch diese Fasern oder andere unter Wärmeeinwirkung kräuselnde Einkomponentenfasem werden zusammen mit anderen Fasern zu einem Vlies abgelegt, das dann aber zur Verfestigung ebenfalls mittels Druck verdichtet werden muss, womit es an Volumen verliert.

Ein anderer Einsatz von Bikomponentenfasem ist in der EP-A-0 171 806 oder 0 171 807 beschrieben. Dort werden die zusammen mit anderen Fasern abgelegten Bikomponentenfasern zur Erzeugung einer ersten Verfestigung mit feinen Wasserstrahlen beaufschlagt und dann das Vliesprodukt auf die Klebe- oder Schmelztemperatur der einen Komponente der Bikomponentenfaser erhitzt, damit diese geschmolzene Komponente sich mit den anderen Fasern fest verbindet, womit eine zweite Verfestigung erzielt wird. Auf diesem Wege wird zwar ein festes Vliesprodukt hergestellt, aber nicht ein voluminöses.

Ein voluminöses und weiterhin flüssigkeitsdurchlässiges Verbundvlies wird in der Hygieneproduktindustrie gebraucht. In Windeln oder anderen Hygieneprodukten muss zunächst ein Flüssigkeitspuffer vorhanden sein, der die Drainage der abgesonderten Körperflüssigkeit in den nachfolgend angeordneten Absorber sowohl in Quer- aber auch und insbesondere in Längsrichtung besorgt. Der Flüssigkeitspuffer muss die abfließende Flüssigkeit schnell aufnehmen und an den sich anschließenden Absorber über dessen ganze Fläche langsam weitergeben.

Der Erfindung liegt die Aufgabe zugrunde ein preiswertes Verfahren zu entwickeln, mit dem ohne Zuhilfenahme von chemischen Bindemitteln wie auch Klebern ein voluminöses und genügend verfestigtes Vlies hergestellt werden kann, das genügend Raum zur schnellen Aufnahme der abgesonderten Flüssigkeit hat und die Flüssigkeit über die Fläche des Vlieses gleichmäßig im Sinne einer Drainage verteilt. Damit hat der folgende Absorber die Möglichkeit die Flüssigkeit langsam und vollständig aus diesem Volumen ab- und aufzusaugen.

Zur Lösung der gestellt Aufgabe sieht die Erfindung vor, dass ein Vlies aus Schrumpffasern zusammen mit nicht schrumpfenden Chemiefasern gebildet, dieses mit einem hydrodynamischen Verfestigungsverfahren ohne Einsatz von Bindemitteln verfestigt, dann das Vlies wärmebehandelt und dabei der in den Schrumpffasern vorhandene Schrumpf durch Temperatureinwirkung ausgelöst wird. Die ausgewählten Schrumpffasern können auch gekräuselte Schrumpf- oder durch Wärmeeinwirkung kräuselnde Bikomponentenfasern sein. Sollte der Schrumpf oder der Kräuselvorgang chemisch ausgelöst werden, so gehört dieser Behandlungsschritt ebenfalls zum Gegenstand der Erfindung. Ein solches Vlies ist durch die Herstellungsart voluminös. Das Volumen nimmt durch die Wasservernadelung nur geringfügig ab, aber durch den Schrumpf- oder Kräuselvorgang der Fasern nimmt das Volumen wieder zu, derart dass das neue Vlies den von den Hygieneprodukten gewünschten Eigenschaften an seinem Einsatzort optimal gerecht wird.

Das Vlies kann aus Stapelfasern und/oder Endlosfasem gebildet werden. Die Schrumpf- und/oder gekräuselten Schrumpf- und/oder kräuselnde Bikomponentenfasern können mit den sich nicht verändernden Fasern vorher bereits vermischt oder aber die jeweilige Schicht der sich unter Wärme verändernden Fasern kann einseitig oder beidseitig aufgetragen werden. Vorteilhaft sind insbesondere Bikomponentenfasern des Typs "side by side" (US-PS 5 108 820), deren eine seitlich an der Faser angelagerte Faserkomponente unter Temperatureinwirkung eine Längenveränderung erfährt. Dadurch wird die Faser insgesamt dreidimensional verformt, das Vlies wird verdichtet und voluminöser. Ähnliches gilt für die reine Schrumpffaser (Monopolymer), die erfindungsgemäß mit nicht schrumpfenden Fasern wasservemadelt ist, so dass nach dem Schrumpfvorgang das ganze Faservlies mehr Volumen in allen Richtungen aufweist. Das Florgewicht kann zwischen 30 und 60 g/m² liegen bei einem Fasertiter von 1,3 - 6,7 dtex.

Eine Vorrichtung zur Durchführung des Verfahrens der erfindungsgemäßen Art ist in der Zeichnung hur schematisch dargestellt. Es zeigen:
- Fig. 1: eine schematisch dargestellte Anlage zur Herstellung eines verfestigten Verbundvlieses aus zwei Faserschichten, womit die Schrumpf- oder Kräuselfaserschicht im Vlies nur einseitig angeordnet ist,
- Fig. 2: die gleiche Anlage wie in Fig. 1, jedoch für ein Vlies mit drei Schichten, wobei die Schrumpf- oder Kräuselfaserschichten auf der Ober- und Unterseite angeordnet sind, und
- Fig. 3: die gleiche Anlage wie in Fig. 1, jedoch für ein Vlies aus nur einer Schicht aus bereits vor der Vlieslegung untereinander vermischten Fasern der genannten Art.

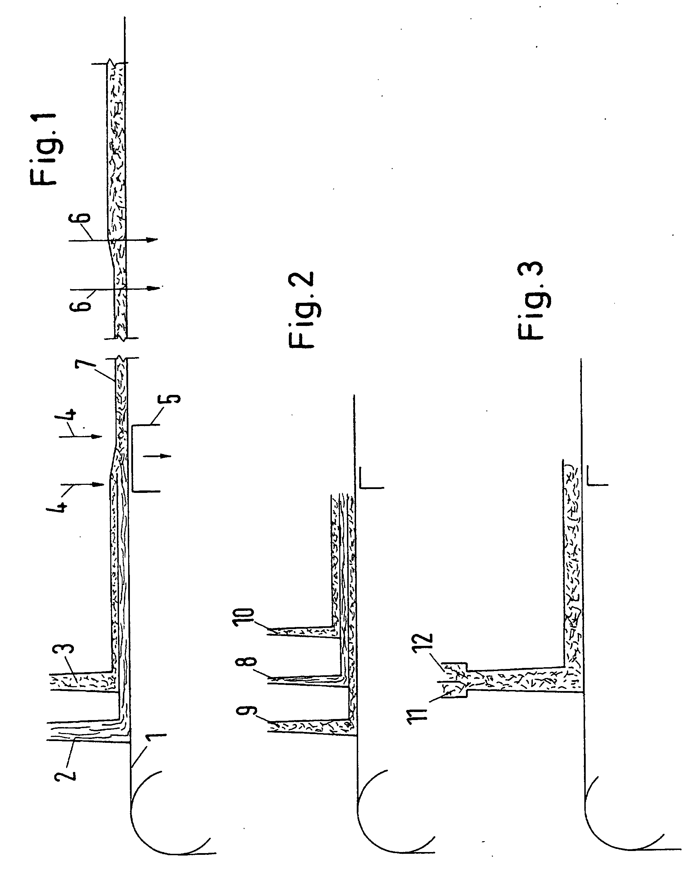

Die Anlage besteht aus mehreren hintereinander aufgestellten Maschinen, die bei kontinuierlicher Arbeitsweise am Ende des Behandlungsverfahrens das gewünschte voluminöse Vlies abliefern. Das mit 1 bezeichnete Endlosband dient zunächst nur zur Bildung des Vlieses und dann zum Transport in und durch die einzelnen Behandlungsmaschinen. Nach Fig. 1 besteht das Vlies aus zwei Schichten, nämlich aus Endlosfasern aus dem Schacht 2 und Stapelfasern aus dem Schacht 3. Die Stapelfasern sollen hier die Schrumpffasem sein. Sie sind damit im wesentlichen nur einseitig in der Vliesstruktur angeordnet.

An die Vlieslegeeinheit schließt sich eine hydrodynamische Vernadelungseinrichtung an, die in der Fig. 1 mit 4 bezeichnet ist. Sie besteht im allgemeinen aus mehreren Düsenbalken, die sich quer über die Arbeitsbreite des Vlieses erstrecken und aus einer Vielzahl von feinen Düsen Wasser mit hohem Druck auf und durch das Vlies spritzen. Dabei werden die einzelnen Fasern des Vlieses untereinander vermischt und verhakt, womit das Vlies ohne Zuhilfenahme von chemischen Bindemitteln verfestigt wird. Das Wasser wird unterhalb des Endlosbandes 1 wieder abgesaugt, was durch den Absaugkasten 5 verdeutlicht sein soll.

Durch den hydraulischen Verfestigungsvorgang ist das vorgelegte Vlies zwar in seinem Volumen vermindert, jedoch schließt sich jetzt der Trocknungsvorgang und der Schrumpf- und/oder Kräuselvorgang der Schrumpf- und/oder Kräuselfasern an. Die Trocknung des Vlieses sollte mit der Warendurchströmung erfolgen, wozu in der Zeichnung die zwei Pfeile 6 gezeichnet sind. Dazu kann das Endlosband 1 durch einen Schachttrockner laufen, es ist aber wirkungsvoller das verfestigte Vlies 7 allein in einen gesondert aufgestellten Trockner wie z. B. einen Siebtrommeltrockner zu führen. In diesem Wärmebehandlungsaggregat erfolgt dann die notwendige Trocknung und ggf. gleich anschließend der Schrumpf- und/oder Kräuselvorgang der vorhandenen Schrumpf- und /oder Kräusel- wie Bikomponentenfaser der besonderen Art. Der Schrumpf- und/oder Kräuselvorgang kann aber auch in einem besonderen, sich anschließenden Schrumpf- und/oder Kräuselofen erfolgen.

Die Anlage in Fig. 2 und 3 ist die gleiche wie in Fig. 1, nur das zu behandelnde Vlies besteht nach Fig. 2 aus drei Schichten und nach Fig. 3 aus nur einer Schicht. Das Vlies nach Fig. 2 besteht aus einer zentralen Endlosfaserschicht 8 auf dessen beiden Seiten Stapelfasern 9, 10 aus Schrumpffasern und/oder Kräusel- wie Bikomponentenfasern der speziellen Art angeordnet sind. In der hydrodynamischen Vernadelungsstation werden die Schichten 8 - 10 miteinander vermischt. Bei der Vliesart nach Fig. 3 werden die unterschiedlichen Fasern 11, 12 bereits bei der Vlieslegung miteinander vermischt. Je nach Anwendungsfall kann das eine oder andere Vlies von Vorteil sein.

## Patentansprüche

1. Verfahren zur Herstellung eines Vlieses aus Kunstfasern wie PES- und/oder Polyolefinfasern vorzugsweise zur Verwendung als Speicherelement für Körperflüssigkeiten im Hygienebereich, **dadurch gekennzeichnet, dass** ein Vlies aus Schrumpffasern zusammen mit nicht schrumpfenden Chemiefasern gebildet, dieses mit einem hydrodynamischen Verfestigungsverfahren ohne Einsatz von Bindemitteln verfestigt, dann das Vlies wärmebehandelt und dabei der in den Schrumpffasern vorhandene Schrumpf durch Temperatureinwirkung ausgelöst wird.

2. Verfahren nach Anspruch 1, **dadurch gekennzeichnet, dass** die Schrumpffasern aus gekräuselten Schrumpffasern bestehen und beim Wärmebehandeln das latent vorhandene Kräuselvermögen ausgelöst wird.

3. Verfahren nach Anspruch 1 oder 2, **dadurch gekennzeichnet, dass** das Vlies aus Endlosfasern gebildet wird.

4. Verfahren nach einem der Ansprüche 1 - 3, **dadurch gekennzeichnet, dass** das Vlies aus Stapelfasern gebildet wird.

5. Verfahren nach einem der Ansprüche 1 - 4, **dadurch gekennzeichnet, dass** das Vlies zumindest teilweise aus unter Temperatureinwirkung kräuselnden Fasern wie insbesondere Bikomponentenfasern des Typs "side by side" hergestellt wird.

6. Verfahren nach einem der Ansprüche 1 - 5, **dadurch gekennzeichnet, dass** die Schrumpffasern bei der Vliesbildung auf einem Vlies aus nicht schrumpfenden Fasern einseitig abgelegt und die beiden Vliesschichten durch die Wasservemadelung auch miteinander verbunden werden.

7. Verfahren nach einem der Ansprüche 1 - 6, **dadurch gekennzeichnet, dass** die Schrumpffasern auf beide Seiten eines Vlieses aus nicht schrumpfenden Fasern gelegt und diese drei Vliesschichten durch die Wasservernadelung auch miteinander verbunden werden.

8. Vorrichtung zur Durchführung eines der Verfahren nach den Ansprüchen 1 - 7, **dadurch gekennzeichnet, dass** die Herstellungsstraße gebildet ist aus einem Vliesleger (2 , 3; 8 - 10; 11, 12) zur Ablage von zumindest teilweise Schrumpffasern, einer Wasservernadelungsvorrichtung (4, 5) zur Verwirbelung und Verschlingung der Fasern des Vlieses, wie bekannt einer Wärmebehandlungs- wie Trocknungsvorrichtung (6), vorzugsweise mit Durchbelüftung, zur Auslösung der latent in den im Vlies vorhandenen Schrumpffasern vorhandenen Schrumpfneigung.

9. Verbundvlies bestehend aus durch Wärmeeinwirkung schrumpfenden Fasern zusammen mit nicht schrumpfenden Chemiefasern, wobei die Fasern als Endlosfasern und/oder Stapelfasern zur Verfügung gestellt sind, wobei das Verbundvlies durch hydrodynamische Vemadelung verfestigt und durch Wärmebehandlung zu einem voluminösen Schrumpfvlies gebildet ist zur Verwendung im Hygienebereich als Docht- bzw. Drainageschicht in Windeln od. dgl.

10. Verbundvlies nach Anspruch 9, **dadurch gekennzeichnet, dass** die schrumpfenden Fasern zumindest teilweise aus kräuselnden Fasern wie insbesondere Bikomponentenfasem des Typs "side by side" bestehen.

## Claims

1. Method of producing a non-woven fabric from synthetic fibres, such as PES-fibres and/or polyolefin fibres, preferably for use as a storage element for body fluids in the hygiene sector, **characterised in that** a non-woven fabric is formed from shrinkable fibres together with non-shrinking chemical fibres, said fabric is strengthened by a hydrodynamic strengthening process without the use of bonding agents, then the non-woven fabric is heat-treated, and the shrinkage, present in the shrinkable fibres, is thereby triggered by the effect of temperature.

2. Method according to claim 1, **characterised in that** the shrinkable fibres comprise crimped shrinkable fibres, and the latent crimpability is triggered during the heat-treatment.

3. Method according to claim 1 or 2, **characterised in that** the non-woven fabric is formed from endless fibres.

4. Method according to one of claims 1 - 3, **characterised in that** the non-woven fabric is formed from stable fibres.

5. Method according to one of claims 1 - 4, **characterised in that** the non-woven fabric is produced, at least partially, from fibres which crimp under the effect of temperature, such as, more especially, bicomponent fibres of the "side by side" type.

6. Method according to one of claims 1 - 5, **characterised in that** the shrinkable fibres are deposited unilaterally on a non-woven fabric formed from non-shrinking fibres during the formation of the non-woven fabric, and the two non-woven fabric layers are also interconnected by the hydraulic needle-punching operation.

7. Method according to one of claims 1 - 6, **characterised in that** the shrinkable fibres are placed on both sides of a non-woven fabric formed from non-shrinking fibres, and said three non-woven fabric layers are also interconnected by the hydraulic needle-punching operation.

8. Apparatus for accomplishing one of the methods according to claims 1 - 7, **characterised in that** the production line is formed from a non-woven fabric layerer (2, 3; 8 - 10; 11, 12) for depositing at least partially shrinkable fibres, an hydraulic needle-punching apparatus (4, 5) for spinning-round and intertwining the fibres of the non-woven fabric, as known, a heat treating and drying apparatus (6), preferably with continuous ventilation, for triggering the tendency to shrink, which is latent in the shrinkable fibres existing in the non-woven fabric.

9. Composite non-woven fabric, comprising fibres which shrink by the effect of heat, together with non-shrinking chemical fibres, wherein the fibres are made available as endless fibres and/or staple fibres, and wherein the composite non-woven fabric is strengthened by hydrodynamic needle-punching and formed by heat treatment to form a bulky shrinkable non-woven fabric for use in the hygiene sector as a capillary or drainage layer in diapers or the like.

10. Composite non-woven fabric according to claim 9, **characterised in that** the shrinking fibres comprise, at least partially, crimping fibres such as, more especially, bicomponent fibres of the "side by side" type.

## Revendications

1. Procédé de fabrication d'un voile à partir de fibres synthétiques comme le polyéthersulfone et / ou des fibres de polyoléfine, le voile étant destiné à être utilisé comme réservoir pour des corps liquides dans le secteur de l'hygiène, et **caractérisé en ce que** un voile est constitué à partir de fibres rétractiles combinées avec des fibres chimiques non rétractiles, **en ce que** ce voile est consolidé par un procédé de consolidation hydrodynamique sans utilisation de colle, **en ce que** ce voile est ensuite traité thermiquement et **en ce que**, lors du traitement thermique, le rétrécissement des fibres rétractiles est provoqué par influence de température.

2. Procédé selon la revendication 1, **caractérisé en ce que** les fibres rétractiles sont composées de fibres rétractiles bouclées et **en ce que** l'effet de bouclage présent de manière latente lors du traitement thermique est déclenché.

3. Procédé selon la revendication 1 ou 2, **caractérisé en ce que** le voile est composé de fibres continues.

4. Procédé selon une des revendications 1 à 3, **caractérisé en ce que** le voile est composé de fibres coupées.

5. Procédé selon une des revendications 1 à 4, **caractérisé en ce que** le voile est fabriqué au moins partiellement à partir de fibres bouclées par effet de température, comme par exemple des fibres de composantes d'image de type 'side by side'.

6. Procédé selon une des revendications 1 à 5, **caractérisé en ce que** lors de la formation du voile, les fibres rétractiles sont disposés d'un même côté sur un voile constitué de fibres rétractiles et **en ce que** les deux couches de voile sont reliées l'une à l'autre par aiguilletage hydraulique.

7. Procédé selon une des revendications 1 à 6, **caractérisé en ce que** les fibres rétractiles sont posées sur les deux côtés d'un voile constitué de fibres non rétractiles et **en ce que** ces trois couches de voile sont également reliées les unes aux autres par aiguilletage hydraulique.

8. Dispositif destiné à réaliser un des procédés selon les revendications 1 à 7, **caractérisé en ce que** le trajet de fabrication est constitué d'un applicateur de voile (2, 3 ; 8-10 ; 11, 12) destiné à déposer des fibres au moins partiellement rétractiles, d'un dispositif d'aiguilletage hydraulique (4, 5) destiné au tourbillonnement et à l'entremêlement des fibres d'un voile et, comme on le sait déjà, d'un dispositif de traitement thermique et de séchage (6), si possible avec aération de part en part afin de provoquer l'effet de rétrécissement présent de manière latente dans les fibres rétractiles elles-mêmes présentes dans le voile.

9. Voile composite constitué de fibres rétractiles sous l'effet de la chaleur et combinées avec des fibres chimiques non rétractiles, les fibres étant mises à disposition sous la forme de fibres continues et / ou de fibres coupées, le voile composite ayant été consolidé par aiguilletage hydrodynamique et ayant été transformé par traitement thermique en un voile rétractile volumineux, tout ceci afin d'être utilisé dans le secteur de l'hygiène comme couche à mèche ou de drainage dans des couches ou analogues.

10. Voile composite selon la revendication 9, **caractérisé en ce que** les fibres rétractiles sont constituées au moins partiellement de fibres bouclées comme par exemple des fibres de composantes d'image de type 'side by side'.
